# EUROPEAN PATENT SPECIFICATION

(11) **EP 0 375 624 B1**
(45) Date of publication and mention of the grant of the patent: **22.02.1995**
(21) Application number: 89810966.5
(22) Date of filing: 19.12.1989
(51) Int. Cl.: A01N 25/18, A01N 33/02, A01N 33/04, A01N 33/08, A01N 43/84

(54) **Low volatility salts**
Salze geringer Flüchtigkeit
Sels à basse volatilité

(30) Priority: 23.12.1988 US 289165
(43) Date of publication of application: 27.06.1990
(73) Proprietor: SANDOZ AG, 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Burns, Johnny Lee, Chicago III. 60643 (US); Wilson, Richard Hansel, Corpus Christi Texas 78413 (US)

(56) References cited:
- DD-A- 161 202
- DD-A- 255 869
- DD-A- 255 870

## Description

This invention relates to the use of various salts to reduce the volatility of biologically active compounds. More specifically, this invention relates to herbicidally active compounds in particular salt forms, the same having reduced volatility as compared with the free-acid form and other salt forms.

It has been found in accordance with this invention that certain amines are particularly useful in varying amounts in reducing the volatility of herbicidally active compounds, which bear a carboxylic acid group, and may even be used for such purpose upon simple addition to other salt forms.

Herbicides which exhibit low volatility are desirable, since herbicides which are subject to drift may not only be damaging to sensitive off-target pests, but may be less effective on target pests.

It has been found that amines selected from the group consisting of
a) Aminopropylmorpholine (APM) as shown by the formula I 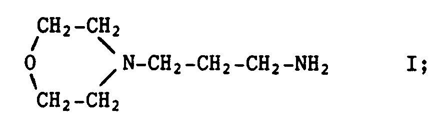
b) Jeffamine D-230 as shown by the formula II 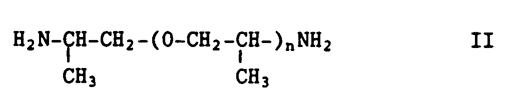 wherein n is 2 or 3;
c) Methyldiethanolamine (MDEA) as shown by the formula III

   CH₃-N-(CH₂-CH₂-OH)₂ III;
d) 2-amino-2-ethyl-1,3-propanediol (AEPD) as shown by the formula IV 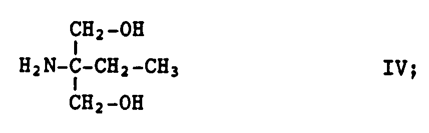
e) Tris(hydroxymethyl)aminomethane (Tris Amino) as shown by the formula V 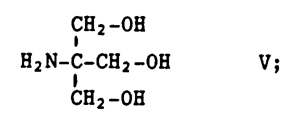 and
f) 2,4,6-Tris(dimethylaminomethyl)phenol (Actiron NX-3) and NaOH as shown by the formula VI 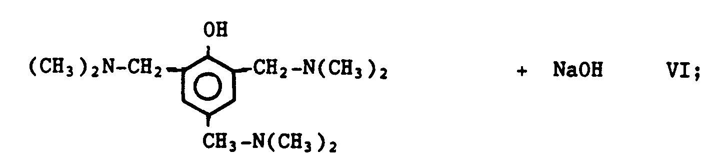 substantially reduce the volatility of herbicidally active compounds without adversely affecting their herbicidal activity. Thus one aspect of this invention is to provide herbicidally active compounds in the aforementioned amine salt form, and mixtures thereof.

The herbicidal compounds whose volatility is reduced in accordance with this invention are the carboxylic acid-containing compounds dicamba (3,6-dichloro-2-methoxy-benzoic acid), and 2,4-D (2,4-dichlorophenoxyacetic acid) salts thereof, and mixtures thereof. A particularly preferred embodiment are the APM salts of dicamba and 2,4-D, and mixtures thereof.

The amine salts of this invention may be made by combining any of the aforementioned amines of Formulae I to VI with the herbicidally active compound, either neatly or with the compound in its available formulation, for example, dry or solid formulations as well as liquid formulations such as aqueous formulations. Also, the amines of Formula I to VI may be added to the pesticidally active compound which is already in another salt form. Such preparations are preferably carried out in aqueous media. For example, an amine of Formula I to VI may be added to dicamba in dimethylamine (DMA) salt form (commercially available from Sandoz Crop Protection Corporation under the trademark BANVEL^{R}) in an aqueous solution at any suitable temperature, e.g., room temperature. In a like manner, the amine of Formula I to VI and a different salt-forming base may be combined with the free acid form of the pesticide. In either case, it is indicated that minor amounts of the amine of Formula I to VI are effective to reduce the volatility of the pesticide in the obtained mixture. Such combination may be used advantageously to reduce the amount of a more expensive amine of Formula I to VI while maintaining greater total salt formulation and low volatility.

The amount of added amine of Formula I to VI may vary widely up to the amount required to neutralize the acid or may be in excess of that amount. The amount of the amine of Formula I to VI may be designated with reference to the amount of that amine required to bring a simple aqueous solution of the pesticide to a pH of 8. For example, if a composition is "1.5X", then it contains 1.5 times the amount of the amine of Formula I to VI necessary to raise the pH to 8. Preferred compositions according to this invention generally contain at least 0.1X, and are preferably in the range of 0.1 to 3X the amount of the compound of Formula I to V, more preferably 0.5 to 2.5X and most preferably 0.5 to 1.5X the amount of the amine of Formula I to VI required.

The herbicides of this invention may be applied and used as pesticides in the same manner and at essentially the same dosages as the parent compounds prior to combination with an amine of Formula I to VI have been used. Somewhat lower dosages may be used in cases where volatility has increased the actual effectiveness of the compound.

The invention is of particular interest for use with compounds effective at combatting weeds, such as dicamba, 2,4-D, or mixtures thereof by applying the amine salt form of this invention in a herbicidally effective amount to the weed plants or their locus.

Generally the amine salts of this invention will be applied at rates depending upon recognized factors such as the specific amine salt, the plants primarily in the locus, the timing, mode and formulation in application, the various conditions of treatment such as soil and weather and the like. However, in general, satisfactory results in weed control are usually obtained upon application of the amine salts of the invention at a rate in the range of from 0.05 to 10 kg/hectare, more usually 0.05 to 2 kg/hectare, and preferably 0.1 to 1 kg/hectare, the application being repeated as necessary. When used in crops, the application usually will not exceed about 5 kg/hectare, and is usually in the range of 0.1 to 2 kg/hectare.

Another aspect of this invention relates to compositions comprising the amine salt of the herbicidally active compound, wherein the amine is selected from the group consisting of the amines of the Formula I to VI as previously defined in combination with an agriculturally inert carrier, either in concentrate form or in dilute form for agricultural application. Concentrates may be in solid form with conventional inert solid carriers and optionally other conventional adjuvants such as wetting agents, sticking agents and the like. Preferably, the compositions are in liquid form and more preferably comprise water as the inert carrier in an amount at least sufficient to dissolve the pesticides of this invention and other salt or free acid form of the compounds present, and excess amines of Formula I to VI which may have been employed, and optionally adjuvants such as wetting agents and the like. Such compositions may also include other pesticides.

Such compositions may contain 0.01% to 99% by weight of the pesticides of this invention, from 0 to 20% by weight of agriculturally acceptable surfactants and 1 to 99.99% by weight of the inert carrier. Higher ratios of surfactant to active ingredient are sometimes desirable and are achieved by incorporation into the formulation or by tank mixing. Application forms of composition typically contain between 0.01 and 25% by weight of the pesticides of this invention, but lower or higher levels can, of course, be present depending on the intended use and the physical properties of the pesticide. Concentrate forms of composition intended to be diluted before use generally contain between 2 and 90%, preferably between 10 and 80% by weight of active ingredient.

Useful compositions or formulations of the compounds of the invention include dusts, granules, pellets, suspension concentrates, wettable powders, emulsifiable concentrates and the like. They are obtained by conventional manner, e.g. by mixing the pesticides of the invention with the inert carrier. More specifically, liquid compositions are obtained by mixing the ingredients, fine solid compositions by blending and, usually grinding, suspensions by wet milling and granules and pellets by impregnating or coating (preformed) granular carriers with the pesticides or by agglomeration techniques.

For example, dusts can be prepared by grinding and blending the pesticide with a solid inert carrier such as talc, clay, silica and the like. Granular formulations can be prepared by impregnating the pesticide, usually dissolved in a suitable solvent, onto and into granulated carriers such as the attapulgites or the vermiculites, usually of a particle size range of from about 0.3 to 1.5 mm. Wettable powders, which can be dispersed in water or oil to any desired concentration of the pesticide, can be prepared by incorporating wetting agents into concentrated dust compositions.

Alternatively, the pesticides of this invention may be used in micro-encapsulated form.

Agriculturally acceptable additives may be employed in the compositions to improve the performance of the pesticide and to reduce foaming, caking and corrosion.

Surfactant as used herein means agriculturally acceptable material which imparts emulsifiability, spreading, wetting, dispersiblity or other surface-modifying properties properties. Examples of surfactants are sodium lignin sulphonate and lauryl sulphate.

Carriers as used herein mean a liquid or solid material used to dilute a concentrated material to a usable or desirable strength. For dusts or granules it can be e.g. talc, kaolin or diatomaeous earth, for liquid concentrate forms, a hydrocarbon such as xylene or an alcohol such as isopropanol; and for liquid application forms, e.g. water or diesel oil.

The compositions of this application can also comprise other compounds having biological activity, e.g. compounds having similar or complementary activity or compounds having antidotal, herbicidal fungicidal or insecticidal activity.

Typical pesticidal composition, according to this invention, are illustrated by the following Examples A, B and C in which the quantities are in parts by weight.

### EXAMPLE A

### Preparation of a Dust

10 Parts of an amine salt according to this invention and 90 parts of powdered talc are mixed in a mechanical grinder-blender and are ground until a homogeneous, free-flowing dust of the desired particle size is obtained. This dust is suitable for direct application to the site of the weed infestation.

### EXAMPLE B

### Preparation of Wettable Powder

25 Parts of an amine salt according to this invention are mixed and milled with 25 parts of synthetic fine silica, 2 parts of sodium lauryl sulphate, 3 parts of sodium ligninsulphonate and 45 parts of finely divided kaolin until the mean particle size is about 5 micron. The resulting wettable powder is diluted with water before use to a spray liquor with the desired concentration.

### EXAMPLE C

### Preparation of Emulsifiable Concentrate (EC)

13.37 Parts of an amine salt according to this invention are mixed in a beaker with 1.43 parts of Toximul 360A (a mixture of anionic and non-ionic surfactants containing largely anionic surfctants), 5.61 parts of Toximul 360A (a mixture of anionic and non-ionic surfactants containing largely non-ionic surfactants), 23.79 parts of dimethylformamide and 55.8 parts of Tenneco 500-100 (predominantly a mixture of alkylated aromatics such as xylene and ethylbenzene) until solution is effected. The resulting EC is diluted with water for use.

The invention may be better illustrated by reference to the following non-limiting examples. Throughout the examples, the term BANVEL^{R} refers to dicamba-DMA.

### EXAMPLE 1

### Compositions

A formulation of each compound is prepared by suspending a known quantity of dicamba and/or 2,4-D in a known quantity of distilled water. This is titrated with the base to a minimum pH of 8.0, recording to amount of base added. Additional water is then added to adjust to the appropriate concentration of active ingredient desired. Typical formulations are given below.

### I. 2,4-D - DMA 439 g/l or 3.7 lb/gal

| | w/w % |
|---|---|
| 2,4D Tech (95% ae) | 41.66 |
| DMA (60%) | 14.45 |
| Distilled water | 43.89 |
| | 1̅0̅0̅.̅0̅0̅ |

### II. Dicamba - DMA 480 g/l or 4.0 lb/gal

| | w/w% |
|---|---|
| Dicamba Tech (80%) | 50.00 |
| DMA (60%) | 17.00 |
| Distilled water | 33.00 |
| | 1̅0̅0̅.̅0̅0̅ |

### IIIa. Dicamba - Tris Amino 480 g/l

| | w/w% |
|---|---|
| Dicamba Tech (90.7%) | 41.19 |
| Tris Amino | 28.05 |
| Distilled water | 30.76 |
| | 1̅0̅0̅.̅0̅0̅ |

### IIIb. 2,4-D Tris Amino

| | w/w% |
|---|---|
| 2,4-D (100%) | 25.00 |
| Tris Amino | 13.70 |
| Distilled water | 61.30 |
| | 1̅0̅0̅.̅0̅0̅ |

### IIIc. Dicamba- 2,4-D - Tris Amino

| | w/w% |
|---|---|
| 2,4-D (100%) | 12.50 |
| Dicamba (100%) | 12.50 |
| Tris Amino | 13.70 |
| Distilled water | 41.95 |
| | 1̅0̅0̅.̅0̅0̅ |

### IIId. Dicamba- 2,4-D - DMA - Tris Amino

| | w/w% |
|---|---|
| 2,4-D (100%) | 12.50 |
| Dicamba (100%) | 12.50 |
| DMA | 5.09 |
| Tris Amino | 13.07 |
| Distilled water | 56.21 |
| | 1̅0̅0̅.̅0̅0̅ |

### IVa. Dicamba - Jeffamine D-230

| | w/w% |
|---|---|
| Dicamba tech (90.7%) | 43.79 |
| Jeffamine D-230 | 24.28 |
| Distilled water | 31.93 |
| | 1̅0̅0̅.̅0̅0̅ |

### IVb. 2,4-D - Jeffamine

| | w/w% |
|---|---|
| 2,4-D (100%) | 25.00 |
| Jeffamine D-230 | 24.77 |
| Distilled water | 50.23 |
| | 1̅0̅0̅.̅0̅0̅ |

### IVc. Dicamba - 2,4-D - Jeffamine

| | w/w% |
|---|---|
| Dicamba (100%) | 12.50 |
| 2,4-D (100%) | 12.50 |
| Jeffamine D-230 | 24.77 |
| Distilled water | 50.23 |
| | 1̅0̅0̅.̅0̅0̅ |

### IVd. Dicamba - 2,4-D - DMA - Jeffamine

| | w/w% |
|---|---|
| Dicamba (100%) | 12.50 |
| 2,4-D (100%) | 12.50 |
| DMA (100%) | 5.09 |
| Jeffamine D-230 | 24.77 |
| Distilled water | 45.24 |
| | 1̅0̅0̅.̅0̅0̅ |

### Va. Dicamba - AEPD

| | w/w% |
|---|---|
| Dicamba tech (90.7%) | 42.85 |
| AEPD | 29.09 |
| Distilled Water | 28.06 |
| | 1̅0̅0̅.̅0̅0̅ |

### Vb. 2,4-D AEPD

| | w/w% |
|---|---|
| 2,4-D (100%) | 25.00 |
| AEPD | 13.48 |
| Distilled water | 61.52 |
| | 1̅0̅0̅.̅0̅0̅ |

### Vc. Dicamba - 2,4-D - AEPD

| | w/w% |
|---|---|
| Dicamba (100%) | 12.50 |
| 2,4-D (100%) | 12.50 |
| AEPD | 13.48 |
| Distilled water | 61.52 |
| | 1̅0̅0̅.̅0̅0̅ |

### Vd. Dicamba - 2,4-D - DMA - AEPD

| | w/w% |
|---|---|
| Dicamba (100%) | 12.50 |
| 2,4-D (100%) | 12.50 |
| DMA (100%) | 5.09 |
| AEPD | 13.48 |
| Distilled water | 56.43 |
| | 1̅0̅0̅.̅0̅0̅ |

### VIa. Dicamba - Actiron NX-3

| | w/w% |
|---|---|
| Dicamba (tech) (89.6%) | 33.87 |
| Actiron NX-3 | 7.67 |
| NaOH (50%) | 7.87 |
| Distilled Water | 50.65 |
| | 1̅0̅0̅.̅0̅0̅ |

### VIb. 2,4-D - Actiron NX-3

| | w/w% |
|---|---|
| 2,4-D (100%) | 25.00 |
| Actiron NX-3 | 7.78 |
| NaOH (50%) | 4.52 |
| Distilled water | 62.70 |
| | 1̅0̅0̅.̅0̅0̅ |

### VIc. Dicamba - 2,4-D Actiron NX-3

| | w/w% |
|---|---|
| Dicamba (100%) | 12.50 |
| 2,4-D (100%) | 12.50 |
| Actiron NK-3 | 7.78 |
| NaOH (50%) | 4.52 |
| Distilled water | 62.70 |
| | 1̅0̅0̅.̅0̅0̅ |

### VId. Dicamba - 2,4-D - DMA - Actiron NX-3

| | w/w% |
|---|---|
| Dicamba (100%) | 12.50 |
| 2,4-D (100%) | 12.50 |
| DMA | 5.09 |
| Actiron NX-3 | 15.56 |
| Distilled water | 54.35 |
| | 1̅0̅0̅.̅0̅0̅ |

### VIIa. Dicamba - MDEA

| | w/w% |
|---|---|
| Dicamba Tech (89.6%) | 23.97 |
| MDEA | 14.41 |
| Distilled water | 61.62 |
| | 1̅0̅0̅.̅0̅0̅ |

### VIIb. 2,4-D - MDEA

| | w/w% |
|---|---|
| 2,4-D (100%) | 25.00 |
| MDEA | 13.48 |
| Distilled water | 61.52 |
| | 1̅0̅0̅.̅0̅0̅ |

### VIIc. Dicamba - 2,4-D - MDEA

| | w/w% |
|---|---|
| Dicamba (100%) | 12.50 |
| 2,4-D (100%) | 12.50 |
| MDEA | 13.48 |
| Distilled water | 61.52 |
| | 1̅0̅0̅.̅0̅0̅ |

### VIId. Dicamba - 2,4-D - DMA - MDEA

| | w/w% |
|---|---|
| Dicamba (100%) | 12.50 |
| 2,4-D (100%) | 12.50 |
| DMA | 5.09 |
| MDEA | 13.48 |
| Distilleded water | 56.43 |
| | 1̅0̅0̅.̅0̅0̅ |

### VIIIa. 2,4-D-APM 230 g/l or 1.9 lb/gal

| | w/w% |
|---|---|
| 2,4-D Tech (95%) | 21.82 |
| APM | 19.41 |
| Distilled water | 58.77 |
| | 1̅0̅0̅.̅0̅0̅ |

### VIIIb. Dicamba-APM 480 g/l or 4 lb/gal

| | w/w% |
|---|---|
| Dicamba Tech (90.7%) | 43.42 |
| APM | 14.17 |
| Distilled water | 42.41 |
| | 1̅0̅0̅.̅0̅0̅ |

### VIIIc. Dicamba-APM 196 g/l or 1.63 lb/gal

| | w/w% |
|---|---|
| Dicamba Tech (90.7%) | 19.67 |
| APM | 18.64 |
| Distilled water | 61.69 |
| | 1̅0̅0̅.̅0̅0̅ |

### VIIId. Dicamba + 2,4-D-APM (1:1 ratio) APM

Dicamba 120 g/l or 1 lb/gal
2,4-D 120 g/l or 1 lb/gal

| | w/w% |
|---|---|
| Dicamba Tech (88%) | 11.70 |
| 2,4-D Tech (95%) | 10.88 |
| APM | 20.16 |
| Distilled water | 57.29 |
| | 1̅0̅0̅.̅0̅0̅ |

### VIIIe. Dicamba + 2,4-D-APM (1:2 ratio) APM

Dicamba 55.2 g/l or 0.46 lb/gal
2,4-D 110.4 g/l or 0.92 lb/gal

| | w/w% |
|---|---|
| Dicamba Tech (88%) | 5.84 |
| 2,4-D Tech (95%) | 10.85 |
| APM | 15.12 |
| Distilled water | 68.19 |
| | 1̅0̅0̅.̅0̅0̅ |

### VIIIf. BANVEL^{R} herbicide + APM

Dicamba 440 g/l or 3.67 lb/gal

| | w/w% |
|---|---|
| Dicamba (83%) | 42.63 |
| DMA (100%) | 10.61 |
| APM | 11.55 |
| Distilled water | 35.21 |
| | 1̅0̅0̅.̅0̅0̅ |

### VIIIg. BANVEL^{R} herbicide + APM

Dicamba 333 g/l or 2.8 lb/gal

| | w/w% |
|---|---|
| Dicamba (83%) | 34.63 |
| DMA (100%) | 8.62 |
| APM | 28.16 |
| Distilled water | 28.59 |
| | 1̅0̅0̅.̅0̅0̅ |

### EXAMPLE 2

### Volatility Studies

Compositions from Example 1 are tested for volatility reduction and compared to technical dicamba. Each of the values below is the estimated half-life based on C¹⁴ measured loss from a petri plate.

| Salt formulation of Dicamba | Half Life (hrs) | Fold Reduction in Volatility |
|---|---|---|
| Technical Dicamba | 36 | ***** |
| Tris Amine | 4101 | 114 |
| AEPD | 6277 | 174 |
| MDEA | 6111 | 170 |
| Actiron NX-3 | no volatility measured | |

### EXAMPLE 3

### Field Studies of Volatility

In this Example and the examples that follow, plot sizes are calculated from meausurements originally made in acres and lbs. according to the conversion factors: 1 hectare = 2.47 acres; 1 kg = 2.2 lbs; 1 m = 3.28 ft; 1 psi = 0.07 kg/sq.cm; 1 gallon = 3.78 l.

Herbicide volatility is measured in a common Bermuda grass pasture with a plot size of 0.04 ha. Application of herbicide is made at a rate of 0.45 kg active ingredient per acre with a CO₂-powered back-pack sprayer and 3.7 m boom containing eight- equally spaced 8004 flat-fan spray nozzles at 2.0 kg/sq.cm pressure and 243 liters of water per ha.

Three Gilian model HFO-113A air sample pumps are placed equal distance downwind to the center of the plots, 3 minutes after completion of the spray application, for a period of 8 hours. Air samples are collected at a rate of 2.6 liters per minute into plastic cassettes containing SKC-227-7 filter pads and are analyzed for herbicide levels using standard gas chromatographic procedures and a Hall detector. The limit of detection is 0.01 »g/sample, and results presented are the average of three measurements.

The studies are performed on days when no rain fell. Temperature was 88°F, and relative humidity 68%. Results are presented below.

| Salt of dicamba | Fold reduction* |
|---|---|
| Tris amine | 17X |
| AEDP | 13X |
| Jeffamine | 12X |
| Actiron NX-3 | 6X |

| | |
|---|---|
| *Reduction is compared with Banvel Herbicide. All salts in this study are titrated to pH 8.0. | |

### EXAMPLE 4

The protocol described in Example 3 is followed to field-test the 2,4-D formulations and dicamba-2,4-D formulations, with the exception that plot sizes vary from 0.04-0.4 ha and application rates vary from 1.1-2.2 kg active ingredient per ha. Temperatures range from 75-91°F and relative humidity from 48-74 %. Results are presented below.

| Herbicide | Rate (kg active ingred. per ha) | Volatility (»g) |
|---|---|---|
| 2,4-D - DMA | 2.2 | 0.29 |
| 2,4-D - APM | 2.2 | 0.01 |
| dicamba - 1.5X DMA | 1.1 | 1.13 |
| dicamba - 1.5X APM | 1.1 | 0.024 |
| BANVEL^{R} | 1.1 | 1.10 |
| BANVEL^{R} + 0.5X APM | 1.1 | 0.08 |
| BANVEL^{R} + 1.4X APM | 1.1 | 0.03 |

### EXAMPLE 5

Effect of Addition of APM to 2,4-D and Dicamba Mixtures.

The following formulations are evaluated using conditions as described in Example 4. Results presented below are the averages of three tests.

| Herbicide | Rate* | Volatility (»g) | | Volatility reduction compared to Dicamba-DMA | Volatility reduction compared to 2,4D-DMA |
|---|---|---|---|---|---|
| | | Dicamba | 2,4D | | |
| BANVEL^{R} | 1.1 | 1.1 | - | - | - |
| 1.5X APM-dicamba | 1.1 | 0.024 | - | 46X | - |
| 2,4-D (DMA) | 2.2 | - | 0.293 | - | - |
| 1.4X APM-dicamba + 2,4-D | 1.1+1.1 | 0.01 | 0.003 | 110X | 97X |
| 1.4X APM-dicamba + 2,4-D | 1.1+2.2 | 0.006 | 0.000 | 183X | Can't measure |

| | | | | | |
|---|---|---|---|---|---|
| * kg active ingredients per ha based on free acid form. | | | | | |

### EXAMPLE 6

### Herbicidal Activity

The effectiveness of the formulations on the control of various weeds in wheat is tested. The various salts of the combination of dicamba-2,4-D are applied at 0.06, 0.125 and 0.25 kg/ha dicamba and 0.38 kg/ha 2,4-D.

Weeds evaluated include:
Amaranthus retroflexus (Pigweed)
Asclepias syriaca (Milkweed)
Brassica campestris (Rape)
Brassica kaber (Charlock)
Descurania pinnata (Tansy mustard)
Descurania sophia (Tansy mustard)
Helianthus annus (Sunflower)
Lactuca serriola (Wild lettuce)
Lamium amplexicaule (Henbit)
Lapsana communis (Nipplewort)
Polygonum convolvulus (Climbing Buckwheat)
Rumex crispus (Yellow dock)
Salsola kali (Russian thistle)
Thlaspi arvenge (Field Penny-Cress)
All of the formulations give substantially the same weed control as DMA formulations, showing that the various salts do not affect activity. Also amount of injury of the various salts is evaluated, and the various salts do not affect the injury rate.

### EXAMPLE 7

Following the protocol of Example 6, weed control and injury rate is evaluated in corn. Weeds which are evaluated include:
Abutilon theophrasti (Velvet-leaf)
Amaranthus retroflexus (Pigweed)
Chenopodium album (Lamb's-quarters)
Polygonum pensylvanicum (Pennsylvania smartweed)
Solanum elaeagnifolium (Silverleaf nightshade)
Xanthium pensylvanicum (Common cocklebur)
No significant differences are seen in either weed control or injury rate compared to DMA formulations.

### EXAMPLE 8

Following the protocol of Example 6, weed control and injury rate is evaluated in sorghum. Weeds which are evaluated include:
Amaranthus retroflexus (Redroot pigweed)
Ipomoea lacunosa (Pitted morningglory)
Polygonum pensylvanicum (Pennsylvania smartweed)
No significant differences are seen in either weed control or injury rate compared to DMA formulations.

## Claims

1. The amine salt of a pesticidally active compound selected from the group consisting of dicamba, 2,4-D, salts thereof, and mixtures thereof, wherein the amine is selected from the group consisting of
a) Aminopropylmorpholine (APM) as shown by the formula I 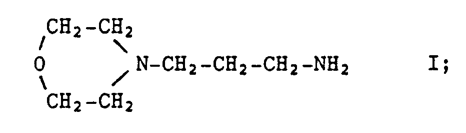
b) Jeffamine D-230 as shown by the formula II 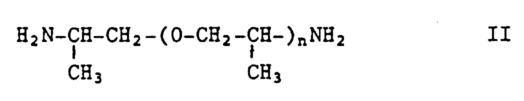 wherein n is 2 or 3;
c) Methyldiethanolamine (MDEA) as shown by the formula III
CH₃-N-(CH₂-CH₂-OH)₂ III;
d) 2-amino-2-ethyl-1,3-propanediol (AEPD) as shown by the formula IV 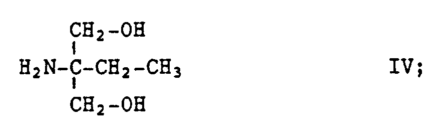
e) Tris(hydroxymethyl)aminomethane (Tris Amino) as shown by the formula V 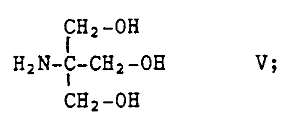 and
f) 2,4,6-Tris(dimethylaminomethyl)phenol (Actiron NX-3) and NaOH as shown by the formula VI

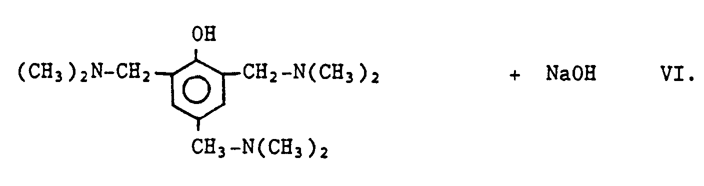

2. The salt of claim 1 having herbicidal activity.

3. The salt of claims 1 or 2 wherein the amine is APM.

4. A pesticidal composition comprising the amine salt of claims 1, 2 or 3 and an agriculturally acceptable carrier.

5. A process for preparing the salt of claims 1, 2 or 3 comprising combining a pesticidally active compound selected from the group consisting of dicamba, 2,4-D, salts thereof, and mixtures thereof with a salt-forming amine selected from the group consisting of
a) Aminopropylmorpholine (APM) as shown by the formula I 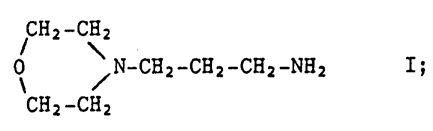
b) Jeffamine D-230 as shown by the formula II 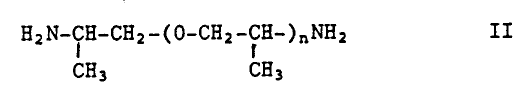 wherein n is 2 or 3;
c) Methyldiethanolamine (MDEA) as shown by the formula III
CH₃-N-(CH₂-CH₂-OH)₂ III;
d) 2-amino-2-ethyl-1,3-propanediol (AEPD) as shown by the formula IV 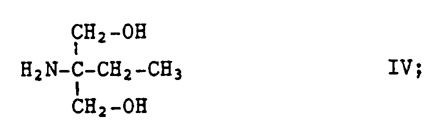
e) Tris(hydroxymethyl)aminomethane (Tris Amino) as shown by the formula V 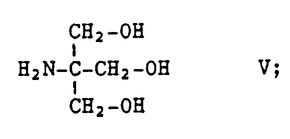 and
f) 2,4,6-Tris(dimethylaminomethyl)phenol and NaOH (Actiron NX-3) and NaOH as shown by the formula VI

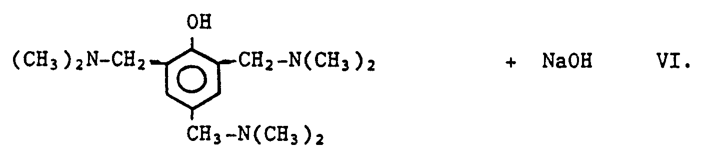

6. A method of combatting weeds comprising applying a herbicidally effective amount of the amine salt of claims 1, 2 or 3 to the weeds of their locus.

## Patentansprüche

1. Aminsalze einer pestizid wirksamen Verbindung ausgewählt aus der Gruppe bestehend aus Dicamba, 2,4-D, Salzen und Mischungen davon, worin das Amin ausgewählt ist aus der Gruppe bestehend aus
a) Aminopropylmorpholin (APM) der Formel I 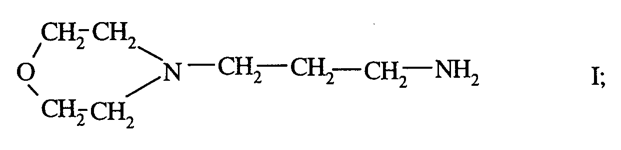
b) Jeffamine D-230 der Formel II 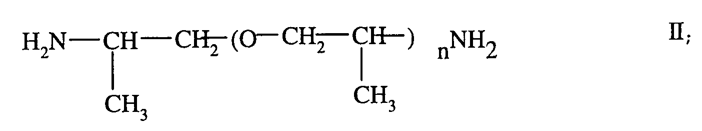 worin n 2 oder 3 ist;
c) Methyldiethanolamin (MDEA) der Formel III
CH₃-N-(CH₂-CH₂-OH)₂ III;
d) 2-Amino-2-ethyl-1,3-propandiol (AEPD) der Formel IV 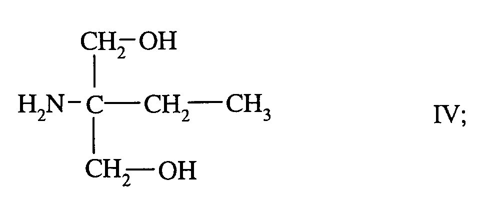
e) Tris(hydroxymethyl)aminomethan (Tris Amino) der Formel V 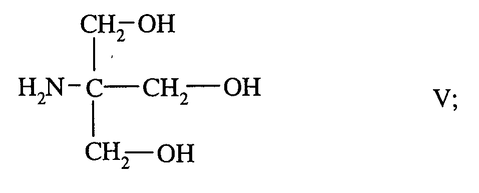 und
f) 2,4,6-Tris(dimethylaminomethyl)phenol (Actiron NX-3) und NaOH gemäss Formel VI

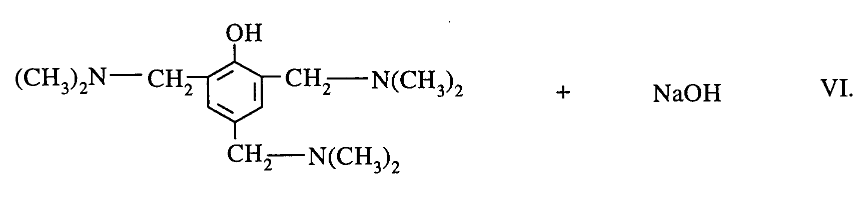

2. Das Salz gemäss Anspruch 1, welches herbizide Aktivität besitzt.

3. Das Salz gemäss Ansprüchen 1 oder 2, worin das Amin APM ist.

4. Ein pestizides Mittel, enthaltend das Aminsalz gemäss Ansprüchen 1, 2 oder 3 und ein in der Landwirtschaft annehmbares Trägermaterial.

5. Verfahren zur Herstellung des Salzes gemäss Ansprüchen 1, 2, oder 3 dadurch gekennzeichnet, dass man eine pestizid wirksame Verbindung ausgewählt aus der Gruppe bestehend aus Dicamba, 2,4-D, Salzen und Mischungen davon mit einem salzbildenden Amin ausgewählt aus der Gruppe bestehend aus
a) Aminopropylmorpholin (APM) der Formel I 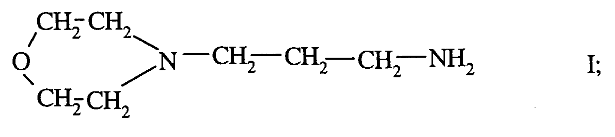
b) Jeffamine D-230 der Formel II 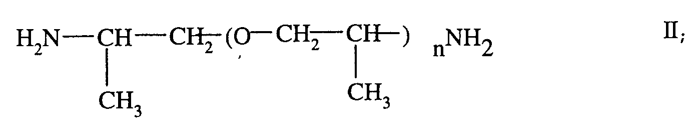 worin n 2 oder 3 ist;
c) Methyldiethanolamin (MDEA) der Formel III
CH₃-N-(CH₂-CH₂-OH)₂ III;
d) 2-Amino-2-ethyl-1,3-propandiol (AEPD) der Formel IV 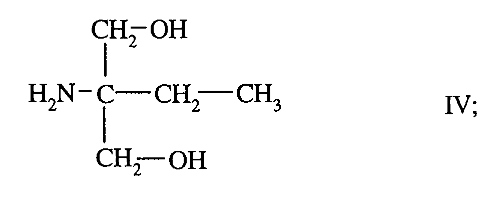
e) Tris(hydroxymethyl)aminomethan (Tris Amino) der Formel V 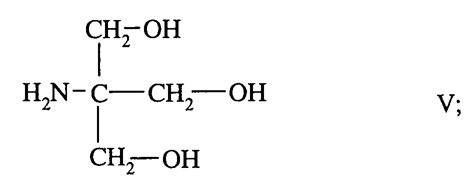 und
f) 2,4,6-Tris(dimethylaminomethyl)phenol (Actiron NX-3) und NaOH gemäss Formel VI 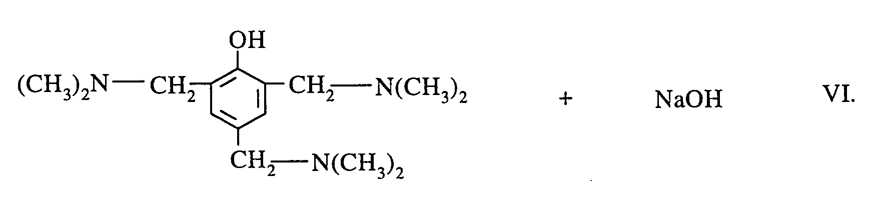 kombiniert.

6. Ein Verfahren zur Bekämpfung von Unkräutern, dadurch gekennzeichnet, dass man eine herbizid wirksame Menge des Aminsalzes gemäss Ansprüchen 1, 2 oder 3 auf die Unkräuter oder ihren Standort appliziert.

## Revendications

1. Le sel d'amine d'un composé actif du point de vue pesticide choisi dans le groupe constitué par le dicamba, le 2,4-D, leurs sels et leurs mélanges, dans lequel l'amine est choisie dans le groupe constitué par
a) l'aminopropylmorpholine (APM) telle que représentée par la formule I 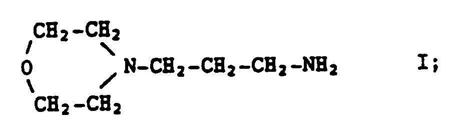
b) la jeffamine D-230 telle que représentée par la formule II 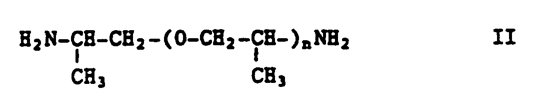 dans laquelle n signifie 2 ou 3,
c) la méthyldiéthanolamine (MDEA) telle que représentée par la formule III
CH₃-N-(CH₂-CH₂-OH)₂ III;
d) le 2-amino-2-éthyl-1,3-propanediol (AEPD) tel que représenté par la formule IV 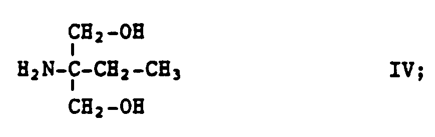
e) le tris(hydroxyméthyl)aminométhane (Tris Amino) tel que représenté par la formule V 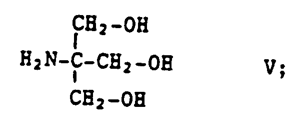 et
f) le 2,4,6-tris(diméthylaminométhyl)phénol (Actiron NX-3) et NaOH tels que représentés par la formule VI

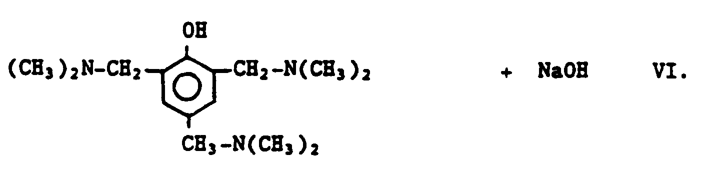

2. Le sel selon la revendication 1 ayant une activité herbicide.

3. Le sel selon la revendication 1 ou 2 dans lequel l'amine est l'APM.

4. Une composition pesticide comprenant le sel d'amine selon la revendication 1, 2 ou 3 et un véhicule acceptable en agriculture.

5. Un procédé de préparation du sel selon la revendication 1, 2 ou 3, comprenant la combinaison d'un composé actif du point de vue pesticide choisi dans le groupe constitué par le dicamba, le 2,4-D, leurs sels et leurs mélanges, avec une amine formant un sel choisie dans le groupe constitué par
a) l'aminopropylmorpholine (APM) telle que représentée par la formule I 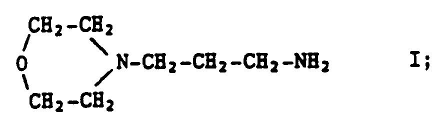
b) la jeffamine D-230 telle que représentée par la formule II 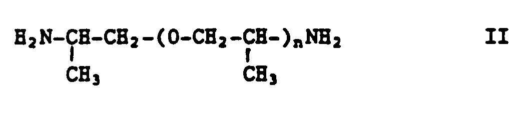 dans laquelle n signifie 2 ou 3,
c) la méthyldiéthanolamine (MDEA) telle que représentée par la formule III
CH₃-N-(CH₂-CH₂-OH)₂ III;
d) le 2-amino-2-éthyl-1,3-propanediol (AEPD) tel que représenté par la formule IV 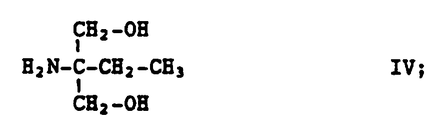
e) le tris(hydroxyméthyl)aminométhane (Tris Amino) tel que représenté par la formule V 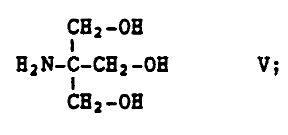 et
f) le 2,4,6-tris(diméthylaminométhyl)phénol (Actiron NX-3) et NaOH tels que représentés par la formule VI

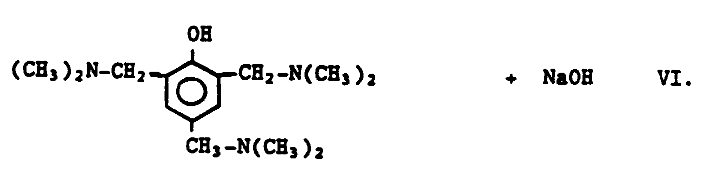

6. Un procédé pour combattre les mauvaises herbes, comprenant l'application sur les mauvaises herbes ou leur zone de croissance d'une quantité efficace du point de vue herbicide du sel d'amine selon la revendication 1, 2 ou 3.
